# EUROPEAN PATENT APPLICATION

(11) **EP 2 965 747 A1**
(43) Date of publication of application: **13.01.2016**
(21) Application number: 14176618.8
(22) Date of filing: 10.07.2014
(51) Int. Cl.: A61K 9/00, A61K 47/02, A61K 47/26, A61K 9/08

(54) **Dialysate acid precursor composition enriched with phosphorous**

(71) Applicant: Nipro Renal Solutions Spain SRL, 25230 Mollerussa (ES)
(72) Inventor: Betriu Bars, Angels, ES-25198 Lleida (ES); Schoenenberger Arnaiz, Juan Antonio, ES-25198 Lleida (ES); Fernandez Giraldez, Elvira, ES-25198 Lleida (ES)
(74) Representative: Gevers Patents

(57) **Abstract**

The invention relates to a dialysate acid precursor composition having a pH less than 4.0 and comprising phosphate in ionic, acid, or organic form, in a water solution, wherein the composition does not comprise methylparaben, neither any one of the compounds selected from saccharin, benzoate, ginger oleoresin, ethanol, and lemon essence.

## Description

### Field of the invention

The present invention relates to the field of extracorporeal blood treatments, and more specifically to renal dialysate compositions.

### Background of the invention

The development of dialysis in its different variants, such as hemodialysis (HD) and peritoneal dialysis (PD) has saved from death many patients who suffer of chronic renal insufficiency. The first treatment units of chronic renal insufficiency through periodical and ambulatory hemodialysis were created in the seventies, however, HD concepts go back to the middle of the nineteenth century, when the Scottish Thomas Graham (1805-1869) described the principles of dialysis in 1854, and defined the movement of several types of solutes through a membrane due to osmotic pressure forces.

When kidneys function correctly, they help the body to keep homeostasis. Kidneys help to reach this balance, eliminating the excess of water and waste metabolic products, as well as keeping the exact levels of pH and electrolytes. Renal insufficiency can be caused by multiple diseases. Hemodialysis is the most common therapeutic measure for an individual whose kidneys cannot carry out its blood purifying function.

A dialysate is the fluid used in dialysis, where the dialysate serves to normalize patients' blood content suffering from kidney disease. During hemodialysis, the blood of the patient is circulated to one side of the dialyzer's membrane, while the dialysate fluid is circulated to the other side of the membrane. The movement of the molecules can occur between the blood and the dialysate thanks across a semi-permeable membrane, which allows the elimination of the waste products and the supplementation of certain elements; these interchange processes between the two compartments are regulated by concentration gradients (diffusion) and by hydrostatic pressure (convection). The diffusion gradient between the blood and the dialysate is established in the dialyzer, between the concentrations of ions in the plasma, and those of the dialysis liquid. The diffusion equilibrium is established between the diffusible fraction in the serum and its concentration in the dialysate. The diffusible fraction corresponds both to the free or ionic forms and to soluble salts, and it does not include the fraction linked to non-diffusible proteins or molecules. Therefore, when a negative balance of a substance is necessary, a lower concentration in the dialysate shall be used in comparison to the concentration in the serum, and vice versa.

The dialysis fluid is one of the fundamental elements of the dialysis treatment, as important as the monitor and the dialyzer. In addition, these three elements interact among them. Thus, it is as important to use a highly biocompatible and high permeability membrane as well as a dialysis solution composition adapted to the patient and free of pyrogens.

Nowadays, hemodialysis is made through an hemodialysis machine that mixes purified water, liquid acid concentrate and liquid or powder bicarbonate concentrate through a mechanism of proportional pumping.

Concentrates are supplied to the dialysis clinics in two ways: generally, the "acid concentrate" is supplied in form of liquid and the bicarbonate is supplied as dry powder. Typically, the acid concentrate includes sodium chloride, calcium chloride, potassium chloride, magnesium chloride, glucose, and sufficient acid (acetic, hydrochloric or citric acid) for the pH equilibrium. The exact composition of the acid concentrate is prescribed by the physician according to the individual needs of the patient at a specific moment..

The dialysis solution made of acid, bicarbonate and the required amount of water is prepared online by the dialysis monitor, and is used immediately to perform the treatment, because the combination of concentrated acid and base solutions can precipitate calcium and magnesium carbonates.

In the field of renal replacement therapy the search for more physiological hemodialysis has introduced new techniques and modalities, mainly to increase depuration of medium-size molecules and inflammation-related factors (hemofiltration, hemodiafiltration or acetate free biofiltration, to name a few). This evolution has made necessary other advances, such as more sophisticated dialysis monitors, more biocompatible dialyzers with a higher ultrafiltration coefficient and very efficient endotoxin filters for dialysis fluid. These changes have thereby increased the cost of hemodialysis, limiting their broad application to patients. However, very few advances have taken place in the area of dialysis fluids in the last decades.

Most patients with chronic kidney disease, especially those patients undergoing hemodialysis, tend to suffer hyperphosphatemia. However certain patients experience hypophosphatemia (defined as a measured plasma inorganic phosphorus fraction lower than 2.6 mg/dL or 0.8 mmol/L), particularly if they suffer from malnutrition or any other predisposing conditions; furthermore some other factors such as aggressive dialysis can induce hypophosphatemia. Phosphate is a primary component of the enzymes and phosphorylated intermediate compounds that performs a key role in the cellular metabolism, in many energy storage and transference as well as in several biological processes of human body.

Currently, there are two available methods to correct hypophosphatemia in hemodialysis patients. On the one hand, intravenous replacement can be performed through continuous perfusion of 1 M monosodium phosphate, monopotassium phosphate, or glycerophosphate, always diluted and infused slowly to prevent acute hypocalcaemia and other clinical complications. On the other hand phosphorus content can also be corrected by adding phosphate-containing compounds to the dialysate.

Both methods make difficult to estimate the real phosphorus balance and to predict the level of hypophosphatemia to be corrected. Thus repeated measures of plasma phosphorus are required. This imprecise prescription often leads to overdosing or infradosing. While infradosing prolongs the hypophosphatemic state, overdosing can produce several complications such as, hypotension, hypocalcemia, cardiac arrhythmias or vascular and metastatic calcifications. It is important to have in mind that intermittent intravenous dosing hampers to maintain a stable plasma phosphate level, since after administration it is eliminated through the hemodialysis process.

The market does not offer any preparation for hemodialysis that includes phosphate ions in its composition. Off-label practices include the enrichment of a liquid, acetic acid-containing 'acid concentrate' and of a liquid 'base concentrate' using soluble sodium phosphate salts to generate phosphorus-rich dialysates. For example, liquid bicarbonate has been mixed with Enema Casen® (a laxative for rectal use, Laboratorios Casen-Fleet, S.L, Utebo, Spain), but currently bicarbonate is available in solid form and hermetically closed and cannot be mixed anymore with said laxative. Others have mixed Enema Casen®, Fleet® Phospho-soda buffered saline laxative, or Fleet® enema (C.B. Fleet Company, Lynchburg, Virginia, USA) with the acid concentrate.

Khan et al., in "Preparation of a Phosphorous-Enriched Hemodialysate Using an 'Acid Concentrate' Solution and a Sodium Bicarbonate Powder", American Journal of Nephrology, 1998; 18; pp. 172-173, describe the addition of phosphate into the acid concentrate of a dual-concentrate, bicarbonate-buffered hemodialysate generating system, by means of the use of a Phospho-soda® buffered saline laxative.

Ing et al., in the review article "Phosphorous-Enriched Hemodialysates: Formulations and Clinical Use", Hemodial International, 2003, 7(2), pp148-155, describe inter alia the enrichment of a liquid, acetic acid-containing 'acid concentrate' and of a liquid 'base carbonate', with the phosphorus preparations Fleet® Phospho-soda buffered saline laxative and Fleet® enema, that contain combinations of monosodium dihydrogen phosphate (NaH₂PO₄·H₂O) and disodium monohydrogen phosphate (Na₂HPO₄·7H₂O).

Su et al., in "Management of hypophosphatemia in nocturnal hemodialysis with phosphate-containing enema: A technical study", Hemodialysis International, 2011; 15, pp. 219-225, disclose the addition of varying amounts of phosphate-containing enema on calcium and phosphate dialysate. Fleet® enema containing monobasic sodium phosphate and bibasic sodium phosphate are mixed with a dialysate acid concentrate.

The technical sheet for Enema Casen®, on its revised version dated June 2004, discloses a composition in which 13.9 g of monohydrated monosodium dihydrogen phosphate (NaH₂PO₄·1H₂O) and 3.2 g of dodecahydrated disodium monohydrogen phosphate (Na₂HPO₄·12H₂O) are mixed together with purified water, methyl parahydroxybenzoate (methylparaben, E 218) and its sodium salt (sodium methylparaben, E 219) for a 100 mL composition.

The technical sheet for Fleet® Phospho-soda buffered oral saline laxative, on its revised version dated June 2003, discloses a 45 mL composition containing 24.4 g of dihydrated monosodium dihydrogen phosphate (NaH₂PO₄·1H₂O) and 10.8 g of dodecahydrated disodium monohydrogen phosphate (Na₂HPO₄·12H₂O), and the excipients glycerol, sodium saccharin, sodium benzoate (E211), ginger aroma (ginger oleoresin, alcohol, lemon essence, lemon essence partially determined, citric acid, water), and purified water. Fleet® enema is reported to include sodium methylhydroxybenzoate as nonmedicinal ingredient.

There are obvious problems deriving from the off-label use of Enema Casen®, Fleet® Phospho-soda buffered saline laxative, and Fleet® enema. These products, which are not approved for dialysis preparation, contain excipients that are known to be allergenic (such as the preservative methylparaben and its sodium salt) or may be toxic for the kidneys, and whose extemporaneous admixture with the dialysate compositions is prone to concentration errors, contamination due to lack of sterility, and many other manipulation errors.

In addition, preparation of acid precursor compositions enriched with phosphate is technically cumbersome due to precipitation problems of the phosphate anions with the calcium and magnesium cations. There is thus a need for a technical solution for the preparation of acid precursor compositions enriched with phosphate which are safe, stable, present no precipitation, have stable concentrations, and are ready-to-use.

In order to solve these problems, the inventors have creatively devised a preparation method of the calcium- and the phosphate-containing solutions, at low pH, low temperatures, and which does not require additional filtration, or if filtration is applied, it does not impact the concentrations of calcium and phosphate because no precipitation of their salts occurs. The prejudice in the art is that the higher the temperature, the higher the solubility. In the present case, with the opposite, i.e. with lower temperatures, higher solubilities are achieved.

There is further a problem in that calcium needs to be administered from 2 to 3.5 mmol/L. With the current state of the art (Su et al.) calcium may only be administered at a maximum concentration of 2 mmol/L.

EP2206504 A1 discloses the preparation of a solution comprising acid concentrate, sodium dihydrogen phosphate (NaH₂PO₄·2H₂O) dissolved in water. The preparation is followed by a filtration step. No pH is indicated but based on the constituents it is calculated to be a neutral pH.

The technical brochure for Phoxilium® (Gambro) describes a renal solution preformulated to prevent hypophosphatemia during continuous renal replacement therapy. This solution comprises bicarbonate and dihydrate phosphate (Na₂HPO₄·2H₂O).

It is an objective of the present invention to provide a dialysate acid precursor composition enriched with phosphate that is safe.

It is another objective of the present invention to provide a dialysate acid precursor composition enriched with phosphate that is stable.

It is a further objective of the present invention to provide a dialysate acid precursor composition enriched with phosphate that is ready-to-use.

It is a further objective of the present invention to provide a dialysate acid precursor composition enriched with phosphate that prevents the manipulation in hospitals of dialysate concentrates and solutions.

It is a further objective of the present invention to provide a dialysate acid precursor composition enriched with phosphate that prevents the intravenous administration of phosphorus and its complications.

It is a further objective of the present invention to provide a dialysate acid precursor composition enriched with phosphate that presents no precipitation.

It is a further objective of the present invention to provide a dialysate acid precursor composition enriched with phosphate that has a stable concentration.

It is an objective of the present invention to provide a dialysate acid precursor composition enriched with phosphate that may comprise calcium concentrations higher than 2 mmol/L, and which can be prepared without precipitation problems.

It is a further objective of the present invention to provide a dialysate acid precursor composition enriched with phosphate that is easy to manufacture.

It is a further objective of the present invention to provide a dialysate acid precursor composition enriched with phosphate that is easy to manufacture.

It is an objective of the present invention to provide a treatment of hypophosphatemia in patients with renal failure.

### Summary of the invention

The present invention is directed to a dialysate acid precursor composition having a pH less than 4.0 and comprising phosphate in ionic, acid, or organic form, in a water solution, characterized in that the composition does not comprise methylparaben; neither any one of the compounds selected from saccharin, benzoate, ginger oleoresin, ethanol, and lemon essence.

The present invention is further directed to a dialysate acid precursor composition having a pH less than 4.0 and comprising phosphate in ionic, acid, or organic form, in a water solution, characterized in that part of the water is the hydration water of a compound selected from NaH₂PO₄·2H₂O, Na₂HPO₄·2H₂O, hydrated α-glycerolphosphate salt, hydrated β-glycerolphosphate salt, and a combination thereof.

The present invention is further directed to a container accommodating the dialysate acid precursor composition.

The present invention is also directed to a buffered dialysate composition comprising the dialysate acid precursor composition according to the present invention, bicarbonate, and additional water, wherein the dialysate acid precursor composition is mixed with bicarbonate, and additional water, in a proportion of 1 part of dialysate acid precursor composition, to 1-2 parts of bicarbonate, to 32-43 parts of water.

The present invention is further directed to a medical device comprising the dialysate acid precursor composition, or the container accommodating said composition, or the buffered dialysate composition of the present invention.

Also, the present invention is directed to a method for the preparation of the dialysate acid precursor composition, said method comprising a) preparing one solution (solution X) comprising from 20 to 80 parts on 100 of the total amount of water, wherein said solution X is a dialysate acid precursor composition as defined herein; b) preparing another solution (solution Y) comprising from 80 to 20 parts on 100 of the total amount of water, wherein said solution Y is a dialysate acid precursor composition as defined herein; wherein a physiologically acceptable acid, magnesium ions, sodium ions, potassium ions, calcium ions, phosphate in ionic, acid, or organic form, and optionally a saccharide, are present in either solution X, solution Y, or both solutions; with the proviso that the calcium and the phosphate are not present in the same solution X or Y; and subsequently c) mixing said solutions X and Y.

The present invention is further directed to the use of disodium hydrogen phosphate dihydrate (Na₂HPO₄·2H₂O), sodium dihydrogen phosphate dihydrate (Na₂HPO₄·2H₂O), α-glycerolphosphate, β-glycerolphosphate, salts, anhydrous and hydrate forms thereof, as a component in a dialysate acid precursor composition.

The present invention is also directed to the use of the dialysate acid precursor composition according to the invention, or the buffered dialysate composition according to the invention, as a medical device.

Also, the present invention is directed to a dialysate acid precursor composition according to the invention, or the buffered dialysate composition according to the invention, for use in therapy; in therapy in a medical device; in treatment of hypophosphatemia in patients with renal failure, for use in hemodialysis (HD) or hemodiafiltration (HDF).

The present invention is further directed to a method of prevention or treatment of hypophosphatemia in patients with renal failure, receiving hemodialysis (HD) or hemodiafiltration (HDF), which comprises administering an effective amount of the dialysate acid precursor composition according to the invention, or the buffered dialysate composition according to the invention, to a patient in need of such prevention or treatment.

The invention will be further elucidated by means of the following description and the appended figures.

### Detailed description of the invention

The present invention relates to a dialysate acid precursor composition having a pH less than 4.0 and comprising phosphate in ionic, acid, or organic form, in a water solution, characterized in that the composition does not comprise methylparaben; neither any one of the compounds selected from saccharin, benzoate, ginger oleoresin, ethanol, and lemon essence.

The present invention relates to a dialysate acid precursor composition having a pH less than 4.0 and comprising phosphate in ionic, acid, or organic form, in a water solution, characterized in that part of the water is the hydration water of a compound selected from NaH₂PO₄·2H₂O, Na₂HPO₄·2H₂O, hydrated α-glycerolphosphate salt, hydrated β-glycerolphosphate salt, and any combination thereof.

The present invention relates to the dialysate acid precursor composition according to any one of the embodiments presented herein, further comprising calcium ions and wherein the molar ratio of total calcium to total phosphate concentration is from 1:2 to 6.5:1.

The present invention relates to the dialysate acid precursor composition according to any one of the embodiments presented herein, wherein the total phosphate concentration is equal to or less than 270 mmol/L.

The present invention relates to the dialysate acid precursor composition according to any one of the embodiments presented herein, wherein the phosphate in ionic, acid, or organic form originates from H₃PO₄, Na₃PO₄, NaH₂PO₄, Na₂HPO₄, KH₂PO₄, K₂HPO₄, K₃PO₄, Mg(H₂PO₄)₂, MgHPO₄, Mg₃(PO₄)₂, Ca(H₂PO₄)₂, CaHPO₄, Ca₃(PO₄)₂, α-glycerophosphoric acid, α-disodium glycerophosphate, α-dipotassium glycerophosphate, α-magnesium glycerophosphate, α-calcium glycerophosphate, β-glycerophosphoric acid, β-disodium glycerophosphate, β-dipotassium glycerophosphate, β-magnesium glycerophosphate, β-calcium glycerophosphate, and anhydrous and hydrate forms thereof, and any combination thereof.

The present invention relates to the dialysate acid precursor composition according to any one of the embodiments presented herein, further comprising calcium at a concentration equal to or less than 157.5 mmol/L.

The present invention relates to the dialysate acid precursor composition according to any one of the embodiments presented herein, wherein the calcium originates from a calcium salt selected from calcium acetate, calcium ascorbate, calcium chloride, calcium citrate, calcium glubionate, calcium gluceptate, calcium gluconate, calcium glycerophosphate, calcium lactate, calcium lactate gluconate, calcium lactobionate, calcium laevulinate, calcium pidolate, calcium sodium lactate, monobasic calcium phosphate, dibasic calcium phosphate, tribasic calcium phosphate, and hydrates thereof, and any combination thereof.

The present invention relates to the dialysate acid precursor composition according to any one of the embodiments presented herein, wherein the composition is at a temperature between 0.1 °C and 40 °C.

The present invention relates to the dialysate acid precursor composition according to any one of the embodiments presented herein, further comprising chloride at a concentration ranging from 2500 to 6000 mmol/L; an acid selected from citric, acetic, malic, fumaric, isocitric, succinic, hydrochloric, oxalic, ascorbic, and lactic acid, a pharmaceutically acceptable salt thereof, and any combination thereof; the citrate ion, if present, at a concentration equal to or less than 75 mmol/L; the acetate ion, if present, at a concentration equal to or less than 360 mmol/L; the lactate, if present, at a concentration equal to or less than 360 mmol/L; and at least one physiologically-acceptable cation different to calcium.

The present invention relates to the dialysate acid precursor composition according to any one of the embodiments presented herein, wherein the at least one physiologically-acceptable cation is selected from hydrogen, sodium at a concentration ranging from 4000 to 7000 mmol/L, potassium at a concentration of equal to or less than 200 mmol/L, and magnesium at a concentration of equal to or less than 55 mmol/L.

The present invention relates to the dialysate acid precursor composition according to any one of the embodiments presented herein, further comprising a saccharide at a concentration of less than 135 g/L.

The present invention relates to the dialysate acid precursor composition according to any one of the embodiments presented herein, wherein the saccharide is selected from glucose, dextrose, glucose monohydrate, polyglucose, polydextrose, fructose, and a polyol selected from glycol, erythritol, threitol, arabitol, xylitol, ribitol, mannitol, sorbitol, galactitol, fucitol, iditol, inositol, volemitol, isomalt, maltitol, lactitol, maltotriitol, maltotetraitol, and polyglycitol.

The present invention relates to a container accommodating the dialysate acid precursor composition according to any one of the embodiments presented herein.

The present invention relates to a buffered dialysate composition comprising i) the dialysate acid precursor composition according to any one of the embodiments presented herein, ii) bicarbonate, and iii) additional water, wherein the i) dialysate acid precursor composition is mixed with ii) bicarbonate, and iii) additional water, in a proportion of 1 part of dialysate acid precursor composition, to 1-2 parts of bicarbonate, to 32-43 parts of water.

The present invention relates to a buffered dialysate composition comprising the dialysate acid precursor composition according to any one of the embodiments presented herein, wherein the bicarbonate has a concentration ranging from 25 to 50 mmol/L.

The present invention relates to a buffered dialysate composition comprising the dialysate acid precursor composition according to any one of the embodiments presented herein, wherein the pH is from 5 to 8.5 at a temperature of 25 °C to 40 °C.

The present invention relates to a medical device comprising the dialysate acid precursor composition according to any one of the embodiments presented herein, or the container according to any one of the embodiments presented herein, or the buffered dialysate composition according to any one of the embodiments presented herein.

The present invention relates to a method for the preparation of the dialysate acid precursor composition, said method comprising a) preparing one solution (solution X) comprising from 20 to 80 parts on 100 of the total amount of water, wherein said solution X is a dialysate acid precursor composition as defined herein; b) preparing another solution (solution Y) comprising from 80 to 20 parts on 100 of the total amount of water, wherein said solution Y is a dialysate acid precursor composition as defined herein; wherein a physiologically acceptable acid, magnesium ions, sodium ions, potassium ions, calcium ions, phosphate in ionic, acid, or organic form, and optionally a saccharide, are present in either solution X, solution Y, or both solutions; with the proviso that the calcium and the phosphate are not present in the same solution X or Y; and subsequently c) mixing said solutions X and Y.

The present invention relates to the method according to any one of the embodiments presented herein, wherein the mixing step is performed under continuous stirring.

The present invention relates to the method according to any one of the embodiments presented herein, wherein the method is carried out at a temperature between 0.1 °C and 20 °C.

The present invention relates to the use of disodium hydrogen phosphate dihydrate (Na₂HPO₄·2H₂O), sodium dihydrogen phosphate dihydrate (NaH₂PO₄·2H₂O), α-glycerolphosphate, β-glycerolphosphate, salts, anhydrous and hydrate forms thereof, as a component in a dialysate acid precursor composition.

The present invention relates to the use of the dialysate acid precursor composition according to any one of the embodiments presented herein, or the buffered dialysate composition according to any one of the embodiments presented herein, as a medical device.

The present invention relates to the dialysate acid precursor composition according to any one of the embodiments presented herein, or the buffered dialysate composition according to any one of the embodiments presented herein, for use in therapy.

The present invention relates to the dialysate acid precursor composition according to any one of the embodiments presented herein, or the buffered dialysate composition according to any one of the embodiments presented herein, for use in therapy in a medical device.

The present invention relates to the dialysate acid precursor composition according to any one of the embodiments presented herein, or the buffered dialysate composition according to any one of the embodiments presented herein, for use in the treatment of hypophosphatemia in patients with renal failure, for use in hemodialysis (HD) or hemodiafiltration (HDF).

The present invention relates to the dialysate acid precursor composition according to any one of the embodiments presented herein, or the buffered dialysate composition according to any one of the embodiments presented herein, for use in the hemodialysis of patients selected from those patients with decreased gastrointestinal absorption of phosphorus, those patients affected by substantial hemodialytic phosphorus removal, those patients with intracellular phosphorus shifts, those patients with severe burns, and those patients with vitamin D deficiency.

The present invention relates to the dialysate acid precursor composition according to any one of the embodiments presented herein, or the buffered dialysate composition according to any one of the embodiments presented herein, for use according to any one of the embodiments presented herein, wherein the patients with decreased gastrointestinal absorption have poor phosphate intake due to anorexia, nausea, vomiting, nasogastric aspiration, alcoholism; or have excessive phosphate-binder therapy.

The present invention relates to the dialysate acid precursor composition according to any one of the embodiments presented herein, or the buffered dialysate composition according to any one of the embodiments presented herein, for use according to any one of the embodiments presented herein, wherein the patients affected by substantial hemodialytic phosphorus removal require intensive hemodialysis or hemodiafiltration and are selected from any patient, pregnant women, hypercatabolic patients, sepsis-induced hypercatabolic patients, patients with uremic pleuritis or pericarditis, patients intoxicated with ethylene glycol, patients intoxicated with methanol, patients intoxicated with lithium, patients intoxicated with vancomycin, patients intoxicated with isopropyl alcohol, patients intoxicated with metformin, and patients intoxicated with any other substance that requires intensive hemodialysis or hemodiafiltration.

The present invention relates to the dialysate acid precursor composition according to any one of the embodiments presented herein, or the buffered dialysate composition according to any one of the embodiments presented herein, for use according to any one of the embodiments presented herein, wherein the patients affected by substantial hemodialytic phosphorus removal requiring intensive hemodialysis or hemodiafiltration, receive 3, 4, 5, or more times per week daily or nocturnal hemodialysis or hemodiafiltration.

The present invention relates to the dialysate acid precursor composition according to any one of the embodiments presented herein, or the buffered dialysate composition according to any one of the embodiments presented herein, for use according to any one of the embodiments presented herein, wherein the patients affected by substantial hemodialytic phosphorus removal are malnourished patients, phosphorus-depleted patients with osteomalacia, non-hyperphosphatemic patients with hypercalcemia, non-hyperphosphatemic patients with hypermagnesemia, or non-hyperphosphatemic patients with ethanol intoxication.

The present invention relates to the dialysate acid precursor composition according to any one of the embodiments presented herein, or the buffered dialysate composition according to any one of the embodiments presented herein, for use according to any one of the embodiments presented herein, wherein the patients with intracellular phosphorus shifts are patients receiving parenteral nutrition, patients with healing of renal osteodystrophy (or hungry bone syndrome), especially after surgical parathyroidectomy, patients undergoing mobilization after prolonged immobilization, or patient with primary respiratory alkalosis like in heat stroke.

The present invention relates to a method of prevention or treatment of hypophosphatemia in patients with renal failure, receiving hemodialysis (HD) or hemodiafiltration (HDF), which comprises administering an effective amount of the dialysate acid precursor composition according to any one of the embodiments presented herein, or the buffered dialysate composition according to any one of the embodiments presented herein, to a patient in need of such prevention or treatment.

The present invention relates to the method according to any one of the embodiments presented herein, wherein the patients are selected from those patients with decreased gastrointestinal absorption of phosphorus, those patients affected by substantial hemodialytic phosphorus removal, those patients with intracellular phosphorus shifts, those patients with severe burns, and those patients with vitamin D deficiency.

The present invention relates to the method according to any one of the embodiments presented herein, wherein the patients with decreased gastrointestinal absorption have poor phosphate intake due to anorexia, nausea, vomiting, nasogastric aspiration, alcoholism; or have excessive phosphate-binder therapy.

The present invention relates to the method according to any one of the embodiments presented herein, wherein the patients affected by substantial hemodialytic phosphorus removal require intensive hemodialysis or hemodiafiltration and are selected from any patient, pregnant women, hypercatabolic patients, sepsis-induced hypercatabolic patients, patients with uremic pleuritis or pericarditis, patients intoxicated with ethylene glycol, patients intoxicated with methanol, patients intoxicated with lithium, patients intoxicated with vancomycin, patients intoxicated with isopropyl alcohol, patients intoxicated with metformin, and patients intoxicated with any other substance that requires intensive hemodialysis or hemodiafiltration.

The present invention relates to the method according to any one of the embodiments presented herein, wherein the patients affected by substantial hemodialytic phosphorus removal requiring intensive hemodialysis or hemodiafiltration, receive 3, 4, 5, or more times per week daily or nocturnal hemodialysis or hemodiafiltration.

The present invention relates to the method according to any one of the embodiments presented herein, wherein the patients affected by substantial hemodialytic phosphorus removal are malnourished patients, phosphorus-depleted patients with osteomalacia, non-hyperphosphatemic patients with hypercalcemia, non-hyperphosphatemic patients with hyperrnagnesemia, or non-hyperphosphatemic patients with ethanol intoxication.

The present invention relates to the method according to any one of the embodiments presented herein, wherein the patients with intracellular phosphorus shifts are patients receiving parenteral nutrition, patients with healing of renal osteodystrophy (or hungry bone syndrome), especially after surgical parathyroidectomy, patients undergoing mobilization after prolonged immobilization, or patient with primary respiratory alkalosis like in heat stroke.

### Definitions and embodiments

Unless otherwise stated or apparent from the context, the term "or" is to be interpreted as inclusive.

The term "dialysate acid precursor" refers to a composition for use during preparation of a ready-for-use dialysis solution or buffered dialysate composition. In order to prepare the ready-for-use dialysis solution, the dialysate acid precursor may be mixed with other components, such as water, a sodium containing concentrate, and a bicarbonate containing concentrate.

The components of the dialysate precursor composition and of the final buffered dialysate composition of the present invention are readily and commercially available. The United States Pharmacopeia (USP) and other equivalents require a degree of purity of at least 95%, approximately. Preferably the degree of purity of all the components is of at least 98 %, more preferably of at least 99 %.

Phosphate in ionic, acid, or organic form includes any compound which can potentially become a phosphate such as any phosphate salt as well as phosphoric acid, since both dissociate into phosphate or hydrogenphosphate (depending of pH) when in solution.

In one embodiment of the present invention, the phosphate in ionic, acid, or organic form originates from H₃PO₄, Na₃PO₄, NaH₂PO₄, Na₂HPO₄, KH₂PO₄, K₂HPO₄, K₃PO₄, Mg(H₂PO₄)₂, MgHPO₄, Mg₃(PO₄)₂, Ca(H₂PO₄)₂, CaHPO₄, Ca₃(PO₄)₂, α-glycerophosphoric acid, α-disodium glycerophosphate, α-dipotassium glycerophosphate, α-magnesium glycerophosphate, α-calcium glycerophosphate, β-glycerophosphoric acid, β-disodium glycerophosphate, β-dipotassium glycerophosphate, β-magnesium glycerophosphate, β-calcium glycerophosphate, and anhydrous and hydrate forms, and any combination thereof.

In one embodiment of the present invention, possible phosphate salts that may be added to the dialysate acid precursor composition of the invention may be tribasic sodium phosphate or trisodium phosphate (Na₃PO₄), monobasic potassium phosphate or potassium dihydrogen phosphate (KH₂PO₄), dibasic potassium phosphate or dipotassium hydrogen phosphate (K₂HPO₄), tribasic potassium phosphate or tripotassium phosphate (K₃PO₄), monobasic magnesium phosphate (Mg(H₂PO₄)₂), dibasic magnesium phosphate (MgHPO₄), tribasic magnesium phosphate (Mg₃(PO₄)₂), monobasic calcium phosphate (Ca(H₂PO₄)₂), dibasic calcium phosphate (CaHPO₄), tribasic calcium phosphate (Ca₃(PO₄)₂).

Organic phosphate salts such as sodium α-glycerolphosphate or sodium β-glycerolphosphate, and hydrates thereof like monohydrate and pentahydrates, are also preferred as they are more soluble in the presence of calcium.

Preferable hydrates are NaH₂PO₄·2H₂O, Na₂HPO₄·2H₂O, NaH₂PO₄·1H₂O, Na₂HPO₄·7H₂O, Na₂HPO₄·12H₂O, monohydrated sodium α-glycerolphosphate, pentahydrated sodium α-glycerolphosphate, monohydrated sodium β-glycerolphosphate, and pentahydrated sodium β-glycerolphosphate. Once dissolved in the composition, the hydration water joins the rest of the water molecules.

"Hydration" refers to the process of adding water molecules to a substance that occurs in a particular solid form and "hydrates" are substances that are formed by adding water molecules. "Solvating" refers to the process of incorporating molecules of a solvent into a substance occurring in a crystalline form. Therefore, the term "solvate" is defined as a crystal form that contains either stoichiometric or non-stoichiometric amounts of solvent. Since water is a solvent, solvates also include hydrates. The term "pseudopolymorph" is applied to polymorphic crystalline forms that have solvent molecules incorporated in their lattice structures. The term pseudopolymorphism is used frequently to designate solvates (Byrn, Pfeiffer, Stowell, (1999) Solid-state Chemistry of Drugs, 2nd Ed. , published by SSCI, Inc).

The term "crystalline" is defined as a form in which the position of the molecules relative to one another is organized according to a three-dimensional lattice structure.

The term "anhydrous form" refers to a particular form essentially free of water.

The water used in the dialysate acid precursor composition of the present invention is dialysis-quality water that meets the standards for use in hemodialysis, such as those standards set forth by AAMI (Association for the Advancement of Medical Instruments), ISO (International Organization for Standardization), ANSI (American National Standards Institute), or the United States or European Pharmacopoeias. These standards are well known by the person skilled in the art, and they establish requirements for purity, with maximum allowable levels of contaminants, bacteria, and endotoxins. Preferably the water is free of pyrogens (i.e. sterile water). A monograph describing water treatment for dialysate, monitoring of water treatment systems, and regulation of water treatment systems is available from AAMI (Standards Collection, Volume 3, Dialysis, Section 3.2 Water Quality for Dialysis, 3 ed., 1998, AAMI, 3330 Washington Boulevard, Arlington, VA 22201) or through the Internet.

The treated water can be obtained using conventional purification techniques, including, for example, distillation, deionization, carbon filtration, microfiltration, ultrafiltration, ultraviolet oxidation, electrodialysis, and reverse osmosis. Additionally, treated water can be bought. Such treated water is used in all, or almost all dialysis clinics, and consequently experts in this technique are familiar with it.

In one embodiment the water solution comprises ultra-pure water (UPW), which is defined as water that has been purified to uncommonly stringent specifications. Typically city feed (or other sources) water may be taken through a series of purification steps that, depending on the quality of UPW wanted, includes gross filtration for large particulates, carbon filtration, water softening, reverse osmosis, exposure to ultraviolet (UV) light for total organic carbon (TOC) and/or bacterial static control, polishing using either ion exchange resins or electro-deionization (EDI) and finally filtration or ultrafiltration. Ultra-pure water has an electrical resistivity of about 18 MΩcm at 25°C. Alternatively, reverse osmosis treated water may be used as the water solution. Reverse osmosis treated water usually has a resistivity ≥ 5 MΩcm.

Ginger oleoresin is defined as is an oil soluble extract from the fruits of *Zingiber Officinale.* Ginger oleoresin may be extracted from ginger rhizome using a solvent such as hexane, or supercritical CO₂.

Lemon essence is defined as a highly concentrated form of pure lemon extract, or a synthetic equivalent thereof. Lemon essence may comprise α-pinene, camphene, b-pinene, sabinene, myrcene, α-terpinene, linalool, b-bisabolene, limonene, trans-a-bergamotene, nerol and neral.

The term "container" refers to a bag, pouch or canister, that may be made of elastic and optionally transparent plastic material, as well as those conventionally employed for accommodating medicinal infusions. The containers may be made of mono-layer film sheets, laminated films, and the like, made of low density polyethylene, ultra-low density polyethylene, high density polyethylene, polypropylene, ethylene-vinyl acetate copolymer, polyester, polyvinyl chloride, polybutadiene, polyamide, ethylene-methacrylate copolymer, ethylene-propylene elastomer, and the like. The film sheets may be prepared by a conventional procedure such as blow molding, inflation molding, T-die molding, multi-layer molding or co-extrusion molding.

The term "buffered dialysate composition" refers herein to a dialysate solution in which the dialysate acid precursor composition of the invention has been mixed with a base concentrate and water, and it is buffered in the sense that it prevents changes in the pH of the resulting solution. The person skilled in the art is familiar with the total equivalents of buffer base available for administration to prepare the final dialysate. Dialysate buffer content is designed to correct the metabolic acidosis that regularly occurs with renal failure.

Base concentrate is currently used in most dialysis clinics. Examples of base concentrates are powdered or liquid sodium bicarbonate. Some base concentrates also contain sodium chloride.The base in a typical base concentrate is bicarbonate, also known as hydrogen carbonate, having the chemical formula HCO₃. Bicarbonate carries a net negative charge, and accordingly will be associated with a positively charged species. Suitable positively charged species include physiologically-acceptable metal cations such as the cationic forms of sodium, potassium, calcium and magnesium. The base from which base concentrate is almost universally prepared in dialysis clinics is sodium bicarbonate, and this is the preferred base in the present compositions and methods. Optionally, the sodium bicarbonate in a base concentrate may be replaced, in part, with a different physiologically-acceptable base. The anionic portion of a suitable replacement for sodium bicarbonate may be, for example, carbonate, lactate, citrate and acetate. Accordingly, the base for a base concentrate may be selected from the salt forms of any of bicarbonate and, optionally, carbonate, lactate, citrate and acetate. Also present in the salt forms will be one or more physiologically-acceptable cations selected from sodium, potassium, calcium and magnesium. These salts and acids are electronically neutral, i.e., there are an equal number of negative and positive charges.

As used herein, "chloride" refers to anionic chloride. Thus, the term "chloride" includes anionic chloride and the salt forms thereof, such as may be formed from chloride anion(s) and physiologically-acceptable cation(s). The term "chloride" is not intended to include compounds wherein the chloride atom is covalently bonded to, for example, a carbon atom in an organic molecule. Exemplary physiologically-acceptable cations include, without limitation, hydrogen ions (i.e., protons), metal cations, and ammonium cations. Metal cations are generally preferred, where suitable metal cations include, but are not limited to, the cationic forms of sodium, potassium, magnesium and calcium. Of these, sodium and potassium are preferred, and sodium is more preferred. A composition containing chloride salts may contain a mixture of physiologically-acceptable cations. Chloride may also be obtained from hydrochloric acid.

The term "citrate" refers to a citrate anion, in any form, salts containing citrate anion, and partial esters of citrate anion. Citric acid, which has been assigned Chemical Abstracts Registry No. 77-92-2, has the molecular formula HOC(CO₂H)(CH₂CO₂H)₂ and a formula weight of 192.12 g/mol. A citrate salt (i.e., a salt containing citrate anion) is composed of one or more citrate anions in association with one or more physiologically-acceptable cations. Exemplary physiologically acceptable cations include, but are not limited to, protons, ammonium cations and metal cations. Suitable metal cations include, but are not limited to, sodium, potassium, calcium, and magnesium, where sodium and potassium are preferred, and sodium is more preferred. A composition containing citrate anion may contain a mixture of physiologically-acceptable cations. A partial ester of a citrate anion will have one or two, but not all three, of the carboxylate (i.e., -COO⁻) groups of citrate anion in an ester form (i.e., -COO-R, where R is an organic group). In addition to one or two R groups, the partial ester of a citrate anion will include one or two physiologically-acceptable cations (so that the total of the R group (s) and cation(s) equals three). The R group is an organic group, preferably a lower alkyl. The citrate is preferably in association with protons and/or metal cations. Exemplary of such citrate compounds are, without limitation, citric acid, sodium dihydrogen citrate, disodium hydrogen citrate, trisodium citrate, trisodium citrate dihydrate, potassium dihydrogen citrate, dipotassium hydrogen citrate, calcium citrate, and magnesium citrate. In one embodiment, the citrate is present in the dialysate acid precursor composition in the form of one or more of citric acid, sodium dihydrogen citrate, disodium hydrogen citrate, potassium dihydrogen citrate, or dipotassium hydrogen citrate. In a preferred embodiment, citric acid provides the source for the citrate anions. In this embodiment, the citric acid functions as the main acidifying agent of the dialysate acid precursor composition. Citric acid is a relatively inexpensive physiological acid that, under ambient conditions, is in the form of a dry chemical powder, crystal, pellet or tablet. Any physiologically tolerable form of citric acid may be used to introduce citrate anions to the composition. For instance, the citric acid may be in the form of a hydrate, including a monohydrate.

Citrate has been previously recognized to be able to function as an anticoagulant in the bloodstream by binding calcium. Accordingly, the citrate concentration of the dialysate precursor composition should be selected in view of its anticoagulant properties. The citrate ion, if present in the dialysate acid precursor composition, should be at a concentration not more than about 75 mmol/L, preferably equal or less than 60 mmol/L, more preferably equal or less than 40 mmol/L, even more preferably equal or less than 30 mmol/L. Most preferably, the concentration of citrate ions in the dialysate acid precursor composition or final dialysate is the one prescribed by the nephrologist.

The term "acetate" refers to an acetate anion, in any form, including salts of acetic acid. Acetate is an organic, monocarboxylate with the formula H₃C-COO⁻. The acetate salt is composed of one or more acetate anions in association with one or more physiologically-acceptable cations. Exemplary physiologically-acceptable cations include, but are not limited to, protons, ammonium cations and metal cations, where metal cations are preferred. Suitable metal cations include, but are not limited to, sodium, potassium, magnesium and calcium, where sodium and potassium are preferred, and sodium is more preferred. Exemplary acetate compounds of the present invention include, but are not limited to, sodium acetate, sodium acetate trihydrate, potassium acetate, calcium acetate, calcium acetate monohydrate, magnesium acetate, and magnesium acetate tetrahydrate. In one embodiment, the acetate of the dialysate acid precursor composition is present in the form of sodium acetate or potassium acetate, and in a preferred embodiment, acetate is in the form of sodium acetate. In another preferred embodiment, acetic acid is present in the dialysate acid precursor composition of the invention optionally combined with an acetate.

The term "lactate" refers to a lactate anion, in any form, including salts of lactic acid. Lactate is an organic, monocarboxylate with the formula H₃C-CH(OH)-COO⁻. A lactate salt is composed of one or more lactate anions in association with one or more physiologically-acceptable cations. Exemplary physiologically-acceptable cations include, but are not limited to, protons, ammonium cations and metal cations, where metal cations are preferred. Suitable metal cations include, but are not limited to, sodium, potassium, magnesium and calcium, where sodium and potassium are preferred, and sodium is more preferred. Exemplary lactate compounds of the present invention include, but are not limited to, lactic acid, sodium lactate, potassium lactate, calcium lactate and magnesium lactate trihydrate. In one embodiment, the lactate of the dialysate acid precursor composition is present in the form of sodium lactate or potassium lactate, and most preferably lactate is in the form of sodium lactate. In another preferred embodiment, lactic acid is present in the dialysate acid precursor composition of the invention optionally combined with a lactate.

In another embodiment of the present invention, the magnesium ion is provided in the form of a magnesium salt selected from the group comprising magnesium chloride, magnesium sulfate, magnesium gluconate and magnesium glubionate.

In another embodiment of the present invention, the dialysate acid precursor composition, in addition to saccharide, may contain one or more amino acids.

The phrase "physiologically-acceptable cations" refers to cations normally found in the blood, plasma, or serum of a mammal, or cations that may be tolerated when introduced into a mammal. Suitable cations include protons, ammonium cations and metal cations. Suitable metal cations include, but are not limited to, the cationic forms of sodium, potassium, calcium, and magnesium, where sodium and potassium are preferred, and sodium is more preferred. An ammonium cation, i.e., a compound of the formula R₄N⁺ where R is hydrogen or an organic group, may be used so long as it is physiologically acceptable. In a preferred embodiment, the cation is selected from hydrogen (i.e., proton), sodium, potassium, calcium, magnesium, and combinations thereof.

The term "medical device" as used herein refers to an instrument, apparatus, implant, in vitro reagent, or similar or related article that is used to prevent or treat disease or other conditions, and does not achieve its purposes through chemical action within or on the body, which would make it a medicinal products, also called pharmaceuticals, that achieve their principal action by pharmacological, metabolic or immunological means. Medical devices act by other means like physical, mechanical, or thermal means, or act outside the human or animal body. An example of medicinal device may be a dialysis apparatus and a dialysate composition.

The terms "continuous stirring" herein refers to a continued movement of agitation. Continuous stirring may be performed in a tank reactor such as a continuous stirred-tank reactor (CSTR).

In general, the concentrations of the components of the dialysate acid precursor composition of the invention are individually prescribed by a physician to reduce, increase, or normalize the concentrations of various components of the patient's blood, plasma, or serum. Accordingly, the precise concentration of these components in the dialysate acid precursor composition, and the buffered dialysate composition prepared therefrom, will be determined by a physician accordingly to principles known in the art.

In an embodiment of the present invention, the dialysate acid precursor composition according to any of the embodiments presented herein may comprise calcium ions wherein the molar ratio of total calcium to total phosphate concentration is of 1.0:2, 1.5:2, 1.0:1, 1.5:1, 2.0:1, 2.5:1, 3.0:1, 3.5:1, 4.0:1, 4.5:1, 5.0:1, 5.5:1, 6.0:1, to 6.5:1.

In an embodiment of the present invention, the total phosphate concentration in the dialysate acid precursor is equal to or less than 270 mmol/L, preferably less than 200 mmol/L, more preferably less than 150 mmol/L, even more preferably less than 100 mmol/L, yet more preferably less than 80 mmol/L, preferably of less than 60 mmol/L, more preferably of less than 50 mmol/L, even more preferably of less than 40 mmol/L. Most preferably, the concentration of phosphate ions in the dialysate acid precursor composition or final dialysate is the one prescribed by the nephrologist

In an embodiment of the present invention, the concentration of sodium ions in the dialysate acid precursor composition ranges from 4000 to 7000 mmol/L, preferably from 4500 to 6500 mmol/L, more preferably being 4865 or 4900 (corresponding to 139x35 and 140x35, respectively) mmol/l for a 1/35 concentrate and 6255 or 6300 (corresponding to 139x45 and 140x45, respectively) for a 1/45 concentrate. Most preferably, the concentration of sodium ions in the dialysate acid precursor composition or final dialysate is the one prescribed by the nephrologist.

In an embodiment of the present invention, the concentration of potassium ions in the dialysate acid precursor composition is equal to or less than 200 mmol/L, preferably less than 150 mmol/L, more preferably between 35.00 and 70.00 mmol/L if 35-fold diluted, also more preferably between 45.00 and 90.00 mmol/L if 45-fold diluted. Most preferably, the concentration of potassium ions in the dialysate acid precursor composition or final dialysate is the one prescribed by the nephrologist.

In another embodiment of the present invention, the concentration of magnesium ions in the dialysate acid precursor composition is equal to or less than 55 mmol/L, preferably of less than 42 mmol/L, more preferably of less than 35 mmol/L, even more preferably of less than 25 mmol/L, preferably 17.50 mmol/L if 35-fold diluted, or 22.50 mmol/L if 45-fold diluted. Most preferably, the concentration of potassium ions in the dialysate acid precursor composition or final dialysate is the one prescribed by the nephrologist.

In another embodiment of the present invention, the concentration of the saccharide in the dialysate acid precursor composition depends on the type of saccharide. If glucose is used, its concentration in the dialysate acid precursor composition is preferably less than 135 g/L, preferably less than 100 g/L, more preferably of less than 60 g/L. Most preferably, the concentration of saccharide and the type of saccharide in the dialysate acid precursor composition or final dialysate is the one prescribed by the nephrologist.

In an embodiment of the present invention, the concentration of chloride ions in the dialysate acid precursor composition ranges from 2500 to 6000 mmol/L, preferably from 3000 to 5500 mmol/L, more preferably from 3100 to 5400 mmol/L. The concentration of chloride ions takes into account the different chloride salts added into the dialysate acid precursor composition.

In another embodiment of the present invention, the concentration of bicarbonate in the buffered dialysate composition ranges from 25 to 50 mmol/L, preferably from 30 to 45 mmol/L, more preferably from 34 to 40 mmol/L. Most preferably, the concentration of bicarbonate in the final dialysate is the one prescribed by the nephrologist.

In a further embodiment of the present invention, the acetate ion is present in the dialysate acid precursor composition at a concentration equal to or less than 360 mmol/L, preferably at a concentration equal or less than 150 mmol/L, more preferably at a concentration equal or less than 100 mmol/L, even more preferably at a concentration equal or less than 50 mmol/L or 10 mmol/L; most preferably the acetate ion is not present in the dialysate acid precursor composition of the invention.

In a further embodiment of the present invention, the lactate ion is present in the dialysate acid precursor composition at concentrations equal to or less than 360 mmol/L, preferably equal or less than 300 mmol/L, more preferably equal or less than 200 mmol/L, even more preferably equal or less than 100 mmol/L, yet more preferably equal or less than 50 mmol/L, preferably equal or less than 1 mmol/L, more preferably equal or less than 0.1 mmol/L; most preferably the lactate ion is not present in the dialysate acid precursor composition of the invention.

In a further embodiment of the present invention, the total calcium concentration in the dialysate acid precursor composition is equal to or less than 157.5 mmol/L, preferably less than 135 mmol/L, which (if 45 fold-diluted) corresponds to a total calcium concentration in the final dialysate of less than 3 mmol/L. Most preferably, the concentration of calcium ions in the dialysate acid precursor composition or final dialysate is the one prescribed by the nephrologist.

The exact dosage and frequency of administration depends on the particular electrolyte and saccharide used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective timely amount may be lowered or increased depending on the response of the treated subject and/or depending on the evaluation of the physician prescribing the compositions of the instant invention. The effective timely amount ranges mentioned hereinabove are therefore only guidelines. The objective is that end stage renal disease (ERSD) patients should restore their physiological values after a dialysis treatment. Thus, while the same concentrate will be therapeutic for some patients and after dialysis their blood electrolytic values will fall into the physiological range, for others patients it may happen that their blood electrolytic values can increase or decrease too much, giving undesirable effects. Alternatively, it can be desired that the dialysis patients receive certain electrolytes (like calcium, potassium) in concentrations that are slightly under or above those generally recommended, because these patients may take a diet or a specific metabolism that substantially impacts their electrolyte physiological concentrations. Accordingly, the nephrologist will adapt the dosage and frequency of administration on a case-by-case basis.

The acid precursor, the bicarbonate and the water are mixed, usually in the dialysis machine, according to different desired dilution proportions. These dilution proportions may be such as provided below, which define the proportion for every 1 part of acid, X parts of bicarbonate (depending of the amount desired in the dialysate), and Y parts of water (remaining parts, until fulfilling the dilution's number). For example, a 1/35 dilution corresponds to 1 part of dialysate acid precursor composition (A), 1.1-1.2 parts of dialysate bicarbonate precursor composition (B), and 32.9- 32.8 parts of water. The total number of parts is 35. A 1/36 dilution corresponds to 1 part of dialysate acid precursor composition (A), 1.6 parts of dialysate bicarbonate precursor composition (B), and 33.4 parts of water. The total number of parts is 36. A 1/45 dilution corresponds to 1 part of dialysate acid precursor composition (A), 1.4-1.543 parts of dialysate bicarbonate precursor composition (B), and 42.6-42.457 parts of water. The total number of parts is 45. "(A)" refers to the acid precursor composition, "(B)" refers to the bicarbonate precursor composition, and "(W)" refers to purified or ultrapure water. The skilled person in the art should note that the above proportions may be varied, and he knows how to adapt the contents accordingly.

In one embodiment of the present invention, the pH of the dialysate acid precursor is preferably comprised between 1.5 and 3.5, more preferably between1.5 and 2.5, at a temperature comprised between 15 °C and 40 °C.

In another embodiment of the present invention, the pH of the final buffered dialysate is comprised between 5.0 and 8.5, preferably between 5.5 and 8, more preferably between 6.0 and 7.5, even more preferably between 7.2 and 7.4, at a temperature comprised between 25 °C and 40 °C.

In one embodiment of the present invention, the final buffered dialysate comprises 0.1-5 mmol/L phosphate ion, 120-150 mmol/L sodium ion, 0-4.5 mmol/L potassium ion, 0-3.5 mmol/L calcium ion, 0-2 mmol/L magnesium ion, 55-135 mmol/L chloride ion, 20-45 mmol/L bicarbonate ion, 0-3 mmol/L citrate ion, 0-3.0 g/l glucose, and water; and does not comprise methylparaben, neither any one of the compounds selected from saccharin, benzoate, ginger oleoresin, ethanol, and lemon essence.

In one embodiment of the present invention, the final buffered dialysate comprises 0.1-5 mmol/L phosphate ion, 120-150 mmol/L sodium ion, 0-4.5 mmol/L potassium ion, 0-3.5 mmol/L calcium ion, 0-2 mmol/L magnesium ion, 55-135 mmol/L chloride ion, 20-45 mmol/L bicarbonate ion, 0-3 mmol/L citrate ion, 0-3.0 g/l glucose, and water; and part of the water is the hydration water of a compound selected from NaH₂PO₄·2H₂O, Na₂HPO₄·2H₂O, hydrated α-glycerolphosphate salt, hydrated β-glycerolphosphate salt, and any combination thereof.

In one embodiment of the present invention, the final buffered dialysate comprises 0.1-5 mmol/L phosphate ion, 120-150 mmol/L sodium ion, 0-4.5 mmol/L potassium ion, 0-3.5 mmol/L calcium ion, 0-2 mmol/L magnesium ion, 55-135 mmol/L chloride ion, 20-45 mmol/L bicarbonate ion, 0-3 mmol/L citrate ion, 0-3.0 g/l glucose, and water; and does not comprise methylparaben, neither any one of the compounds selected from saccharin, benzoate, ginger oleoresin, ethanol, and lemon essence; and part of the water is the hydration water of a compound selected from NaH₂PO₄·2H₂O, Na₂HPO₄·2H₂O, hydrated α-glycerolphosphate salt, hydrated β-glycerolphosphate salt, and any combination thereof.

### Methods of preparation

Suitable sources for the ingredients of the compositions of the present invention are well known in the art. Indeed, the chemical characteristics for the compounds used in the present invention, such as molecular weight and solubility, are available in the art such that one of ordinary skill in the art will know how to prepare the composition of the present invention. See, e.g., the Sigma and Aldrich catalogs from Sigma-Aldrich. All of these chemicals are commercially available, in pharmaceutical grade if desired, from many chemical supply houses.

The present invention relates to a method for the preparation of a dialysate acid precursor composition according to any one of the embodiments presented herein, said method comprising
a) preparing one solution (solution X) comprising from 20 to 80 parts on 100 of the total amount of water, wherein said solution X is the dialysate acid precursor composition as defined in any one of the embodiments presented herein;
b) preparing another solution (solution Y) comprising from 80 to 20 parts on 100 of the total amount of water, wherein said solution Y is the dialysate acid precursor composition as defined in any one of the embodiments presented herein;
   wherein a physiologically acceptable acid, magnesium ions, sodium ions, potassium ions, calcium ions, phosphate in ionic, acid, or organic form, and optionally a saccharide, are present in either solution X, solution Y, or both solutions;
   with the proviso that the calcium and the phosphate are not present in the same solution X or Y; and subsequently
c) mixing said solutions X and Y.

In a further embodiment, the method for the preparation of a dialysate acid precursor composition according to the invention, comprises
a) dissolving the required quantities of citric acid and/or acetic acid, optionally a saccharide, a magnesium salt, a sodium salt, a potassium salt, and a calcium salt in 20 to 80 parts on 100 of the total amount of water; the salt preferably being a chloride salt;
b) dissolving the required quantity of a compound generating phosphates in ionic and acid form in 80 to 20 parts on 100 of the total amount of water; and
c) mixing the solutions obtained from steps a) and b).

In another embodiment, the method for the preparation of a dialysate acid precursor composition according to the invention, comprises
a) dissolving the required quantities of citric acid and/or acetic acid, optionally a saccharide, a magnesium salt, a sodium salt, a potassium salt, and a compound generating phosphates in ionic and acid form in 20 to 80 parts on 100 of the total amount of water; the salt preferably being a chloride salt; the salt preferably being a chloride salt;
b) dissolving the required quantity of calcium salt in 80 to 20 parts on 100 of the total amount of water; the salt preferably being a chloride salt; and
c) mixing the solutions obtained from steps a) and b).

In one embodiment, in the method for the preparation of a dialysate acid precursor composition, the compound generating phosphate in ionic, acid, or organic form is phosphoric acid, Na₂HPO₄, NaH₂PO₄·2H₂O, α-glycerolphosphate, β-glycerolphosphate, or any combination thereof.

The acid can be added to one solution or both. If added to one solution, it can be added to solution X or Y, and the solutions X and Y are mixed.

The person skilled in the art will note that steps a) and b) may be performed in the reverse order, thus first step b) and then step a). What is important is that the calcium ions and the phosphate in ionic, acid, or organic form are not present in the same starting solutions X or Y. In other words, the calcium salts and the phosphate acid or salts are firstly dissolved in separate solutions.

When adding the physiologically acceptable acid in both solutions, the acid (for example citric and/or acetic acid) should be added considering the proportion of the total amount water used in the solution (e.g. 80% parts of total amount of water, require 80% of total amount of required acid). As described above, alternatives to the citric and/or acetic acid may also be used, like malic, fumaric, isocitric, succinic, hydrochloric, oxalic, ascorbic, and lactic acid, pharmaceutically acceptable salts thereof, and any combination thereof.

The optional saccharide, the magnesium salt, the sodium salt, and the potassium salt can be added in both calcium and phosphate solutions. They can be split and be shared out in both solutions.

In one embodiment, the mixing in step c) is performed under continuous stirring.

In another embodiment, the method is carried out at a temperature between 0.1 °C and 20 °C.

### Applications in the medical field

The present invention relates to the use of the dialysate acid precursor composition according to any one of the embodiments presented herein, or to the use of the buffered dialysate composition according to any one of the embodiments presented herein, for the manufacture of a medicament for the prevention or treatment of hypophosphatemia in patients with renal failure, for use in hemodialysis (HD) or hemodiafiltration (HDF).

The present invention relates to the use of the dialysate acid precursor composition according to any one of the embodiments presented herein, or to the use of the buffered dialysate composition according to any one of the embodiments presented herein, for the manufacture of a medicament for the hemodialysis of patients selected from those patients with decreased gastrointestinal absorption of phosphorus, those patients affected by substantial hemodialytic phosphorus removal, those patients with intracellular phosphorus shifts, those patients with severe burns, and those patients with vitamin D deficiency.

The present invention relates to the use of the dialysate acid precursor composition according to any one of the embodiments presented herein, or to the use of the buffered dialysate composition according to any one of the embodiments presented herein, for the manufacture of a medicament for the prevention or treatment of patients with decreased gastrointestinal absorption having poor phosphate intake due to anorexia, nausea, vomiting, nasogastric aspiration, alcoholism; or have excessive phosphate-binder therapy.

The present invention relates to the use of the dialysate acid precursor composition according to any one of the embodiments presented herein, or to the use of the buffered dialysate composition according to any one of the embodiments presented herein, for the manufacture of a medicament for the prevention or treatment of patients affected by substantial hemodialytic phosphorus removal and require intensive hemodialysis or hemodiafiltration and are selected from any patient, pregnant women, hypercatabolic patients, sepsis-induced hypercatabolic patients, patients with uremic pleuritis or pericarditis, patients intoxicated with ethylene glycol, patients intoxicated with methanol, patients intoxicated with lithium, patients intoxicated with vancomycin, patients intoxicated with isopropyl alcohol, patients intoxicated with metformin, and patients intoxicated with any other substance that requires intensive hemodialysis or hemodiafiltration.

The present invention relates to the use of the dialysate acid precursor composition according to any one of the embodiments presented herein, or to the use of the buffered dialysate composition according to any one of the embodiments presented herein, for the manufacture of a medicament for the prevention or treatment of patients affected by substantial hemodialytic phosphorus removal and requiring intensive hemodialysis or hemodiafiltration, which receive 3, 4, 5, or more times per week daily or nocturnal hemodialysis or hemodiafiltration.

The present invention relates to the use of the dialysate acid precursor composition according to any one of the embodiments presented herein, or to the use of the buffered dialysate composition according to any one of the embodiments presented herein, for the manufacture of a medicament for the prevention or treatment of patients affected by substantial hemodialytic phosphorus removal that are malnourished patients, phosphorus-depleted patients with osteomalacia, non-hyperphosphatemic patients with hypercalcemia, non-hyperphosphatemic patients with hypermagnesemia, or non-hyperphosphatemic patients with ethanol intoxication.

The present invention relates to the use of the dialysate acid precursor composition according to any one of the embodiments presented herein, or to the use of the buffered dialysate composition according to any one of the embodiments presented herein, for the manufacture of a medicament for the prevention or treatment of patients having intracellular phosphorus shifts, and are patients receiving parenteral nutrition, patients with healing of renal osteodystrophy (or hungry bone syndrome), especially after surgical parathyroidectomy, patients undergoing mobilization after prolonged immobilization, or patients with primary respiratory alkalosis like in heat stroke.

In a further embodiment of the present invention, the dialysate acid precursor composition may be used in patients on nocturnal hemodialysis, long hemodialysis, short daily hemodialysis.

### Examples

### Example 1: Preparation of an acid precursor containing citric acid and sodium acetate (Solution A) with a pH of 2.3

The solution A of acid precursor was prepared under refrigeration since low temperatures increase solubility of calcium phosphate salts. 5L of acid solution were prepared with the required quantities of citric acid, acetic acid, glucose, magnesium chloride and potassium chloride. The required quantity of calcium chloride was dissolved in 2L of the acid solution prepared in the first step. The required quantity of sodium dihydrogen phosphate was dissolved in 2L of the acid solution prepared in the first step.

These two solutions were then mixed under continuous stirring. The final solution A was achieved by completing the obtained 4L solution to a final 5L volume with the remaining 1L acid concentrate of the first step. No calcium phosphate precipitates were observed. The constituents of Solution A and their concentrations are listed in the following Table 1, which provides quantities per 1000 mL.

**Table 1**

| Compound | Mass (g) | Molecular weight (g/mol) | Anion concentration (mmol/L) | Cation concentration (mmol/L) |
|---|---|---|---|---|
| NaCl Sodium Chloride | 200.84 | 58.44 | Cl⁻ : 3436.7 | Na⁺ 3436.7 |
| NaH₂PO₄ · 2 H₂O Sodium dihydrogen phosphate dihydrate | 5.05 | 156.01 | PO₄³⁻: 32.4 | Na⁺: 32.4 |
| MgCl₂·6H₂O Magnesium chloride | 3.38 (anhydrous 1.80) | 202.4 (anhydrous: 94.4) | Cl⁻: 33.4 | Mg²⁺: 16.7 |
| KCI Potassium Chloride | 3.91 | 74.55 | Cl⁻: 52.4 | K⁺: 52.4 |
| C₆H₈O₇ Citric acid | 5.38 | 192 | C₆H₇O₇⁻:28 | H⁺: 28 |
| C₆H₁₂O₆ Glucose | 38.50 | 180 | 213.9 | |
| CaCl₂·2H₂O Calcium Chloride | 7.72 (anhydrous: 5.836) | 147.01 | Cl⁻: 105 | Ca²⁺: 52.5 |
| NaC₂H₃O₂-3H₂O Sodium acetate | 1.43 (anhydrous: 0.86) | 135.99 (anhydrous 81.99) | C₂H₃O₂⁻: 10.5 | Na⁺:10.5 |

Total ions per 1000 mL: Na⁺ 3479.7 mmol; K⁺ 52.4 mmol; Ca⁺⁺ 52.5 mmol; Mg⁺⁺ 16.7 mmol; Phosphate 32.4 mmol; chloride 3627.5 mmol.

The dialysate resulting from the use of solution A with a base concentrate had the following ionic composition: Na 140 mEq/L; Ca 3mEq/L; P 3 mg/dL (PO₄³⁻: 0.9 mmol/L); K 1.5 mEq/L; Mg 0.95 mEq/L.

### Example 2: Preparation of an acid precursor containing acetic acid but no citric acid (Solution B) with a pH of 2.5

The solution B of acid precursor was prepared under refrigeration since low temperatures increase solubility of calcium phosphate salts. 5L of acid solution were prepared with the needed quantities of acetic acid, glucose, magnesium chloride and potassium chloride. The needed quantity of calcium chloride was dissolved in 2L of the acid solution prepared in the first step. The needed quantity of sodium dihydrogen phosphate was dissolved in 2L of the acid solution prepared in the first step.

These two solutions were then mixed under continuous stirring. The final solution B was achieved by completing the obtained 4L solution to a final 5L volume with the remaining 1L acid concentrate of the first step. No calcium phosphate precipitates were observed. The constituents of Solution B and their concentrations are listed in the following Table 2, which provides quantities per 1000 mL.

**Table 2**

| Compound | Mass (g) | Molecular weight (g/mol) | Anion concentration (mmol/L) | Cation concentration (mmol/L) |
|---|---|---|---|---|
| NaCl Sodium Chloride | 201.9 | 58.44 | Cl⁻ : 3454.8 | Na⁺: 3454.8 |
| CaCl₂·2H₂O Calcium Chloride | 7.73 | 147.01 | Cl⁻ : 105.2 | Ca²⁺: 52.6 |
| NaH₂PO₄·2H₂O Sodium dihydrogen phosphate dihydrate | 5.04 | 156.01 | PO₄³⁻: 32.3 | Na⁺ : 32.3 |
| MgCl₂·6H₂O Magnesium chloride | 3.37 | 202.4 | Cl⁻: 33.4 | Mg²⁺ : 16.7 |
| KCI Potassium Chloride | 4.95 | 74.55 | Cl⁻: 66.4 | K⁺ : 66.4 |
| C₆H₁₂O₆ Glucose | 57.75 | 180 | 320.8 | |
| C₂H₃O₂ Acetic acid | 5.38 | 60.05 | C₂H₂O₂⁻:89 | H⁺ : 89 |

| | | | | |
|---|---|---|---|---|
| Total ions per 1000 mL: Na⁺ 3487.1 mmol; K⁺ 66.4 mmol; Ca²⁺ 52.6 mmol; Mg²⁺ 16.7 mmol; Phosphate 32.3 mmol; chloride 3659.8 mmol. | | | | |

The dialysate resulting from the use of solution B with a base concentrate had the following ionic composition: Na⁺ 140 mEq/L; Ca²⁺ 3mEq/L; P 3 mg/dL (PO₄³⁻: 0.9 mmol/L); K⁺ 1.9 mEq/L; Mg²⁺ 0.95 mEq/L.

### Example 3: Preparation of an acid precursor containing phosphoric acid and sodium citrate, but no acetic acid (Solution C) with a pH of 2.5

The solution C of acid precursor was prepared under refrigeration since low temperatures increase solubility of calcium phosphate salts. 4L of acid solution were prepared with the needed quantities of NaCl, phosphoric acid, disodium hydrogen phosphate, sodium citrate, glucose, magnesium chloride and potassium chloride. 1 L of a water solution containing the needed quantity of calcium chloride was prepared. These two solutions were then mixed under continuous stirring. No calcium phosphate precipitates were observed. The constituents of Solution C and their concentrations are listed in the following Table 3, which provides quantities per 1000 mL.

**Table 3**

| Compound | Mass (g) | Molecular weight (g/mol) | Anion concentration (mmol/L) | Cation concentration (mmol/L) |
|---|---|---|---|---|
| NaCl Sodium Chloride | 201.25 | 58.44 | Cl⁻: 3443.7 | Na⁺: 3443.7 |
| CaCl₂·2H₂O Calcium Chloride | 7.72 | 147.01 | Cl⁻ : 105 | Ca²⁺: 52.5 |
| Na₂HPO₄ disodium hydrogen phosphate (anhydrous) | 1.85 | 142 | PO₄³⁻: 13 | Na⁺: 26 |
| H₃PO₄ Phosphoric acid | 1.9 | 97.97 | PO₄³⁻: 19.4 | |
| MgCl₂·6H₂O Magnesium chloride | 3.38 | 202.4 | Cl⁻: 33.4 | Mg²⁺ : 16.7 |
| KCI Potassium Chloride | 4.95 | 74.55 | Cl⁻: 66.4 | K⁺ : 66.4 |
| C₃H₅O(COO)₃ Na₃·2 H₂O Sodium Citrate | 0.81 | 192 | C₃H₅O(COO)₃ : 4.2 | Na⁺: 12.6 |
| C₆H₁₂O₆ Glucose | 38.85 | 180 | 215.8 | |

| | | | | |
|---|---|---|---|---|
| Total ions per 1000 mL: Na⁺ 3482.3 mmol; K⁺ 66.4 mmol; Ca²⁺ 52.5 mmol; Mg²⁺ 16.7 mmol; Phosphate 32.4 mmol; chloride 3648.5 mmol. | | | | |

The dyalisate resulting from the use of solution C with a base concentrate had the following ionic composition: Na⁺ 140 mEq/L; Ca²⁺ 3mEq/L; P 3 mg/dL (PO₄³⁻: 0.9 mmol/L); K⁺ 1.9 mEq/L; Mg²⁺ 0.95 mEq/L.

### Example 4: Preparation of an acid precursor containing citric acid and sodium acetate (Solution A-Gluc) with a pH of 2.3

The solution A-Gluc of acid precursor was prepared under refrigeration since low temperatures increase solubility of calcium phosphate salts. 4L of acid solution were prepared with the needed quantities of sodium chloride, sodium phosphate, acetic acid, glucose, magnesium chloride and potassium chloride. 1 L of a water solution containing the needed quantity of calcium gluconate was prepared. These two solutions were than mixed under continuous stirring. No calcium phosphate precipitates were observed. The constituents of Solution A-Gluc and their concentrations are listed in the following Table 4, which provides quantities per 1000 mL.

**Table 4**

| Compound | Mass (g) | Molecular weight (g/mol) | Anion concentration (mmol/L) | Cation concentration (mmol/L) |
|---|---|---|---|---|
| NaCl Sodium Chloride | 200.84 | 58.44 | Cl⁻ : 3436.7 | Na⁺: 3436.7 |
| NaH₂PO₄ ·2 H₂O Sodium dihydrogen phosphate dihydrate | 5.05 | 156.01 | PO₄³⁻: 32.4 | Na⁺ : 32.4 |
| MgCl₂·6H₂O Magnesium chloride | 3.38 (anhydrous 1.80) | 202.4 (anhydrous 94.4) | Cl⁻: 33.4 | Mg²⁺: 16.7 |
| KCI Potassium Chloride | 3.91 | 74.55 | Cl⁻: 52.4 | K⁺: 52.4 |
| C₆H₈O₇ Citric Acid | 5.38 | 192 | C₆H₇O₇⁻: 28 | |
| C₆H₁₂O₆ Glucose | 38.50 | 180 | 213.8 | |
| C₁₂H₂₂CaO₁₄ Calcium Gluconate | 22.5 | 430.37 | | Ca²⁺: 52.3 |
| NaC₂H₃O₂·3H₂O | 1.43 (anhydrous: 0.86) | 135.99 (anhydrous 81.99) | C₂H₃O₂⁻: 10.5 | Na⁺: 10.5 |

| | | | | |
|---|---|---|---|---|
| Total ions per 1000 mL: Na⁺ 3479.6 mmol; K⁺ 52.4 mmol; Ca⁺⁺ 52.3 mmol; Mg⁺⁺ 16.7 mmol; Phosphate 32.4 mmol; chloride 3522.5 mmol. | | | | |

The dyalisate resulting from the use of solution A-Gluc with a base concentrate had the following ionic composition: Na 140 mEq/L; Ca 3mEq/L; P 3 mg/dL (PO₄³⁻: 0.9 mmol/L); K 1.5 mEq/L; Mg 0.95 mEq/L.

### Example 5: Preparation of an acid precursor containing acetic acid but no citric acid (Solution B-Gluc) with a pH of 2.5

The solution B-Gluc of acid precursor was prepared under refrigeration since low temperatures increase solubility of calcium phosphate salts. 4L of acid solution were prepared with the needed quantities of sodium chloride, sodium phosphate, acetic acid, glucose, magnesium chloride and potassium chloride. 1 L of a water solution containing the needed quantity of calcium gluconate was prepared. These two solutions were then mixed under continuous stirring. No calcium phosphate precipitates were observed. The constituents of Solution B-Gluc and their concentrations are listed in the following Table 5, which provides quantities per 1000 mL.

**Table 5**

| Compound | Mass (g) | Molecular weight (g/mol) | Anion concentration (mmol/L) | Cation concentration (mmol/L) |
|---|---|---|---|---|
| NaCl Sodium Chloride | 201.9 | 58.44 | Cl⁻ : 3454.8 | Na⁺ :3454.8 |
| NaH₂PO₄·2 H₂O Sodium dihydrogen phosphate dihydrate | 5.04 | 156.01 | PO₄³⁻: 32.3 | Na⁺ : 32.3 |
| MgCl₂·6H₂O Magnesium chloride | 3.37 | 202.4 | Cl⁻: 33.4 | Mg²⁺ : 16.7 |
| KCl Potassium Chloride | 4.95 | 74.55 | Cl⁻: 66.4 | K⁺ : 66.4 |
| C₁₂H₂₂CaO₁₄ Calcium gluconate | 22.5 | 430.37 | | Ca²⁺: 52.3 |
| C₆H₁₂O₆ Glucose | 57.75 | 180 | 220.8 | |
| C₂H₃O₂ Acetic acid | 5.38 | 60.05 | C₂H₂O₂⁻: 89.6 | |

| | | | | |
|---|---|---|---|---|
| Total ions per 1000 mL: Na⁺ 3487.1 mmol; K⁺ 66.4 mmol; Ca²⁺ 52.3 mmol; Mg²⁺ 16.7 mmol; Phosphate 32.3 mmol; chloride 3554.6mmol. | | | | |

The dyalisate resulting from the use of solution B-Gluc with a base concentrate had the following ionic composition: Na⁺ 140 mEq/L; Ca²⁺ 3mEq/L; P 3 mg/dL (PO₄³⁻: 0.9 mmol/L); K⁺ 1.9 mEq/L; Mg²⁺ 0.95 mEq/L.

### Example 6: Preparation of an acid precursor containing phosphoric acid and sodium citrate but no acetic acid (C-Gluc) with a pH of 2.5

The solution C-Gluc of acid precursor was prepared under refrigeration since low temperatures increase solubility of calcium phosphate salts. 4L of acid solution were prepared with the needed quantities of sodium chloride, phosphoric acid, glucose, magnesium chloride and potassium chloride. 1 L of a water solution containing the needed quantity of calcium gluconate was prepared. These two solutions were then mixed under continuous stirring. No calcium phosphate precipitates were observed. The constituents of Solution C-Gluc and their concentrations are listed in the following Table 6, which provides quantities per 1000 mL.

**Table 6**

| Compound | Mass (g) | Molecular weight (g/mol) | Anion concentration (mmol/L) | Cation concentration (mmol/L) |
|---|---|---|---|---|
| NaCl Sodium Chloride | 201.25 | 58.44 | Cl⁻ : 3443.7 | Na⁺: 3443.7 |
| C₁₂H₂₂CaO₁₄ Calcium gluconate | 22 | 430.37 | | Ca²⁺: 51.1 |
| Na₂HPO₄ disodium hydrogen phosphate (anhydrous) | 1.85 | 142 | P0₄³⁻: 13 | Na²⁺ : 26 |
| H₃PO₄ Phosphoric acid | 1.9 | 97.97 | PO₄³⁻: 19.4 | |
| MgCl₂·6H₂O Magnesium chloride | 3.38 | 202.4 | Cl⁻: 33.4 | Mg²⁺ : 16.7 |
| KCl Potassium Chloride | 4.95 | 74.55 | Cl⁻: 66.4 | K⁺ : 66.4 |
| C₃H₅O(COO)₃Na₃· 2 H₂O Sodium Citrate | 0.81 | 192 | C₃H₅O(COO)₃: 4.2 | Na⁺: 12.6 |
| C₆H₁₂O₆ Glucose | 38.85 | 180 | 215.8 | |

| | | | | |
|---|---|---|---|---|
| Total ions per 1000 mL: Na⁺ 3482.3 mmol; K⁺ 66.4 mmol; Ca²⁺ 51.1 mmol; Mg²⁺ 16.7 mmol; Phosphate 32.4 mmol; chloride 3543.5 mmol. | | | | |

The dialysate resulting from the use of solution C-Gluc with a base concentrate had the following ionic composition: Na⁺ 140 mEq/L; Ca²⁺ 3mEq/L; P 3 mg/dL (PO₄³⁻: 0.9 mmol/L); K⁺ 1.9 mEq/L; Mg²⁺ 0.95 mEq/L.

## Claims

1. A dialysate acid precursor composition having a pH less than 4.0 and comprising phosphate in ionic, acid, or organic form, in a water solution, **characterized in that** the composition does not comprise:
- methylparaben;
- neither any one of the compounds selected from saccharin, benzoate, ginger oleoresin, ethanol, and lemon essence.

2. A dialysate acid precursor composition having a pH less than 4.0 and comprising phosphate in ionic, acid, or organic form, in a water solution, **characterized in that** part of the water is the hydration water of a compound selected from NaH₂PO₄·2H₂O, Na₂HPO₄·2H₂O, hydrated α-glycerolphosphate salt, hydrated β-glycerolphosphate salt, and any combination thereof.

3. The dialysate acid precursor composition according to claim 1 or 2, wherein the total phosphate concentration is equal to or less than 270 mmol/L.

4. The dialysate acid precursor composition according to any one of the preceding claims, wherein the phosphate in ionic, acid, or organic form originates from H₃PO₄, Na₃PO₄, NaH₂PO₄, Na₂HPO₄, KH₂PO₄, K₂HPO₄, K₃PO₄, Mg(H₂PO₄)₂, MgHPO₄, Mg₃(PO₄)₂, Ca(H₂PO₄)₂, CaHPO₄, Ca₃(PO₄)₂, α-glycerophosphoric acid, α-disodium glycerophosphate, α-dipotassium glycerophosphate, α-magnesium glycerophosphate, α-calcium glycerophosphate, α-glycerophosphoric acid, α-disodium glycerophosphate, α-dipotassium glycerophosphate, β-magnesium glycerophosphate, β-calcium glycerophosphate, and anhydrous and hydrate forms thereof, and any combination thereof.

5. The dialysate acid precursor composition according to any one of the preceding claims, further comprising calcium at a concentration equal to or less than 157.5 mmol/L.

6. The dialysate acid precursor composition according to claim 5, wherein the calcium originates from a calcium salt selected from calcium acetate, calcium ascorbate, calcium chloride, calcium citrate, calcium glubionate, calcium gluceptate, calcium gluconate, calcium glycerophosphate, calcium lactate, calcium lactate gluconate, calcium lactobionate, calcium laevulinate, calcium pidolate, calcium sodium lactate, monobasic calcium phosphate, dibasic calcium phosphate, tribasic calcium phosphate, and hydrates thereof, and any combination thereof.

7. The dialysate acid precursor composition according to any one of the preceding claims, wherein the composition is at a temperature between 0.1 °C and 40 °C.

8. The dialysate acid precursor composition according to any one of the preceding claims, further comprising chloride at a concentration ranging from 2500 to 6000 mmol/L; an acid selected from citric, acetic, malic, fumaric, isocitric, succinic, hydrochloric, oxalic, ascorbic, and lactic acid, a pharmaceutically acceptable salt thereof, and any combination thereof; the citrate ion, if present, at a concentration equal to or less than 75 mmol/L; the acetate ion, if present, at a concentration equal to or less than 360 mmol/L; the lactate, if present, at a concentration equal to or less than 360 mmol/L; and at least one physiologically-acceptable cation different to calcium.

9. The dialysate acid precursor composition according to claim 8, wherein the at least one physiologically-acceptable cation is selected from hydrogen, sodium at a concentration ranging from 4000 to 7000 mmol/L, potassium at a concentration of equal to or less than 200 mmol/L, and magnesium at a concentration of equal to or less than 55 mmol/L.

10. The dialysate acid precursor composition according to any one of the preceding claims, further comprising a saccharide at a concentration of less than 135 g/L.

11. The dialysate acid precursor composition according to claim 10, wherein the saccharide is selected from glucose, dextrose, glucose monohydrate, polyglucose, polydextrose, fructose, and a polyol selected from glycol, erythritol, threitol, arabitol, xylitol, ribitol, mannitol, sorbitol, galactitol, fucitol, iditol, inositol, volemitol, isomalt, maltitol, lactitol, maltotriitol, maltotetraitol, and polyglycitol.

12. A container accommodating the dialysate acid precursor composition according to any one of the preceding claims.

13. A buffered dialysate composition comprising i) the dialysate acid precursor composition according to any one of claims 1 to 11, ii) bicarbonate, and iii) additional water, wherein the i) dialysate acid precursor composition is mixed with ii) bicarbonate, and iii) additional water, in a proportion of 1 part of dialysate acid precursor composition, to 1-2 parts of bicarbonate, to 32-43 parts of water.

14. The buffered dialysate composition according to claim 13, wherein the bicarbonate has a concentration ranging from 25 to 50 mmol/L.

15. The buffered dialysate composition according to claim 13 or 14, wherein the pH is from 5 to 8.5 at a temperature of 25 °C to 40 °C.

16. A medical device comprising the dialysate acid precursor composition according to any one of claims 1 to 11, or the container according to claim 12, or the buffered dialysate composition according to any one of claims 13 to 15.

17. A method for the preparation of a dialysate acid precursor composition, said method comprising
a) preparing one solution (solution X) comprising from 20 to 80 parts on 100 of the total amount of water, wherein said solution X is as defined in claim 9;
b) preparing another solution (solution Y) comprising from 80 to 20 parts on 100 of the total amount of water, wherein said solution Y is as defined in claim 9;
wherein a physiologically acceptable acid, magnesium ions, sodium ions, potassium ions, calcium ions, phosphate in ionic, acid, or organic form, and optionally a saccharide, are present in either solution X, solution Y, or both solutions;
with the proviso that the calcium and the phosphate are not present in the same solution X or Y; and subsequently
c) mixing said solutions X and Y.

18. The method according to claim 17, wherein the mixing in step c) is performed under continuous stirring.

19. The method according to claim 17 or 18, wherein the method is carried out at a temperature between 0.1 °C and 20 °C.

20. Use of disodium hydrogen phosphate dihydrate (Na₂HPO₄·2H₂O), sodium dihydrogen phosphate dihydrate (NaH₂PO₄·2H₂O), α-glycerolphosphate, β-glycerolphosphate, salts, anhydrous and hydrate forms thereof, as a component in a dialysate acid precursor composition.

21. Use of the dialysate acid precursor composition according to any one of claims 1-11, or the buffered dialysate composition according to any one of claims 13-15, as a medical device.

22. The dialysate acid precursor composition according to any one of claims 1-11, or the buffered dialysate composition according to any one of claims 13-15, for use in therapy.

23. The dialysate acid precursor composition according to any one of claims 1-11, or the buffered dialysate composition according to any one of claims 13-15, for use in therapy in a medical device.

24. The dialysate acid precursor composition according to any one of claims 1-11, or the buffered dialysate composition according to any one of claims 13-15, for use in the treatment of hypophosphatemia in patients with renal failure, for use in hemodialysis (HD) or hemodiafiltration (HDF).

25. The dialysate acid precursor composition according to any one of claims 1-11, or the buffered dialysate composition according to any one of claims 13-15, for use in the hemodialysis of patients selected from those patients with decreased gastrointestinal absorption of phosphorus, those patients affected by substantial hemodialytic phosphorus removal, those patients with intracellular phosphorus shifts, those patients with severe burns, and those patients with vitamin D deficiency.

26. The dialysate acid precursor composition according to any one of claims 1-11, or the buffered dialysate composition according to any one of claims 13-15, for use according to claim 25, wherein the patients with decreased gastrointestinal absorption have poor phosphate intake due to anorexia, nausea, vomiting, nasogastric aspiration, alcoholism; or have excessive phosphate-binder therapy.

27. The dialysate acid precursor composition according to any one of claims 1-11, or the buffered dialysate composition according to any one of claims 13-15, for use according to claim 25, wherein the patients affected by substantial hemodialytic phosphorus removal require intensive hemodialysis or hemodiafiltration and are selected from any patient, pregnant women, hypercatabolic patients, sepsis-induced hypercatabolic patients, patients with uremic pleuritis or pericarditis, patients intoxicated with ethylene glycol, patients intoxicated with methanol, patients intoxicated with lithium, patients intoxicated with vancomycin, patients intoxicated with isopropyl alcohol, patients intoxicated with metformin, and patients intoxicated with any other substance that requires intensive hemodialysis or hemodiafiltration.

28. The dialysate acid precursor composition according to any one of claims 1-11, or the buffered dialysate composition according to any one of claims 13-15, for use according to claim 27, wherein the patients affected by substantial hemodialytic phosphorus removal requiring intensive hemodialysis or hemodiafiltration, receive 3, 4, 5, or more times per week daily or nocturnal hemodialysis or hemodiafiltration.

29. The dialysate acid precursor composition according to any one of claims 1-11, or the buffered dialysate composition according to any one of claims 13-15, for use according to claim 25, wherein the patients affected by substantial hemodialytic phosphorus removal are malnourished patients, phosphorus-depleted patients with osteomalacia, non-hyperphosphatemic patients with hypercalcemia, non-hyperphosphatemic patients with hypermagnesemia, or non-hyperphosphatemic patients with ethanol intoxication.

30. The dialysate acid precursor composition according to any one of claims 1-11, or the buffered dialysate composition according to any one of claims 13-15, for use according to claim 25, wherein the patients with intracellular phosphorus shifts are patients receiving parenteral nutrition, patients with healing of renal osteodystrophy (or hungry bone syndrome), patients with hungry bone syndrome after surgical parathyroidectomy, patients undergoing mobilization after prolonged immobilization, or patient with primary respiratory alkalosis like in heat stroke.

31. A method of prevention or treatment of hypophosphatemia in patients with renal failure, receiving hemodialysis (HD) or hemodiafiltration (HDF), which comprises administering an effective amount of the dialysate acid precursor composition according to any one of claims 1-11, or the buffered dialysate composition according to any one of claims 13-15, to a patient in need of such prevention or treatment.

32. The method according to claim 31, wherein the patients are selected from those patients with decreased gastrointestinal absorption of phosphorus, those patients affected by substantial hemodialytic phosphorus removal, those patients with intracellular phosphorus shifts, those patients with severe burns, and those patients with vitamin D deficiency.

33. The method according to claim 32, wherein the patients with decreased gastrointestinal absorption have poor phosphate intake due to anorexia, nausea, vomiting, nasogastric aspiration, alcoholism; or have excessive phosphate-binder therapy.

34. The method according to claim 32, wherein the patients affected by substantial hemodialytic phosphorus removal require intensive hemodialysis or hemodiafiltration and are selected from any patient, pregnant women, hypercatabolic patients, sepsis-induced hypercatabolic patients, patients with uremic pleuritis or pericarditis, patients intoxicated with ethylene glycol, patients intoxicated with methanol, patients intoxicated with lithium, patients intoxicated with vancomycin, patients intoxicated with isopropyl alcohol, patients intoxicated with metformin, and patients intoxicated with any other substance that requires intensive hemodialysis or hemodiafiltration.

35. The method according to claim 34, wherein the patients affected by substantial hemodialytic phosphorus removal requiring intensive hemodialysis or hemodiafiltration, receive 3, 4, 5, or more times per week daily or nocturnal hemodialysis or hemodiafiltration.

36. The method according to claim 32, wherein the patients affected by substantial hemodialytic phosphorus removal are malnourished patients, phosphorus-depleted patients with osteomalacia, non-hyperphosphatemic patients with hypercalcemia, non-hyperphosphatemic patients with hypermagnesemia, or non-hyperphosphatemic patients with ethanol intoxication.

37. The method according to claim 32, wherein the patients with intracellular phosphorus shifts are patients receiving parenteral nutrition, patients with healing of renal osteodystrophy (or hungry bone syndrome), patients with hungry bone syndrome after surgical parathyroidectomy, patients undergoing mobilization after prolonged immobilization, or patient with primary respiratory alkalosis like in heat stroke.
